# EUROPEAN PATENT APPLICATION

(11) **EP 2 062 524 A1**
(43) Date of publication of application: **27.05.2009**
(21) Application number: 07767012.3
(22) Date of filing: 31.05.2007
(51) Int. Cl.: A61B 1/04, G02B 23/24

(54) **ENDOSCOPE DEVICE**

(30) Priority: 12.09.2006 JP 2006247168
(71) Applicant: Olympus Medical Systems Corp., Tokyo 151-0072 (JP)
(72) Inventor: NIIDA, Koichi, Tokyo 1510072 (JP)
(74) Representative: von Hellfeld, Axel
(86) International application number: PCT/JP2007/061090
(87) International publication number: WO 2008/032478

(57) **Abstract**

An endoscope apparatus includes: a first video signal generating portion that generates a first video signal displayed on a display device as an endoscope image from an image pickup signal picked up by an image pickup device of an endoscope; and a second video signal generating portion that performs an image processing of the first video signal in accordance with application software that runs on an operating system and generates a second video signal. The endoscope apparatus includes: a video signal selecting portion that selects one of the first video signal and the second video signal and outputs the signal to the display device; and a control portion that determines or estimates whether the second signal generating portion can output the second video signal, and applies a control signal so that the video signal selecting portion outputs the first video signal when determining or estimating that the second signal generating portion cannot output the second video signal.

## Description

### Technical Field

The present invention relates to an endoscope apparatus that generates an endoscope image by a signal processing using application software that runs on an operating system.

### Background Art

In medical fields, observation has been widely performed using image pickup apparatuses such as an X-ray diagnostic apparatus, a CT, an MRI, an ultrasound observation apparatus, and an endoscope apparatus.

As an endoscope apparatus among the image pickup apparatuses, an electronic endoscope system is disclosed in, for example, Japanese Patent Application Laid-Open Publication No. 2001-218735.

The electronic endoscope system includes an insertion portion that can be inserted into a body cavity, and picks up an image of a target in the body cavity formed by an objective optical system placed in a distal end portion of the insertion portion, with image pickup means such as a solid-state image pickup device, and outputs the image as an image pickup signal.

The electronic endoscope system has a configuration and an operation for displaying an image of the target in the body cavity on display means such as a monitor on the basis of the image pickup signal. A user such as an operator observes, for example, an organ in the body cavity on the basis of the picked-up image of the inside of the body cavity displayed on the display means such as the monitor.

As disclosed in the electronic endoscope system in the publication, the image pickup signal from the image pickup means of the endoscope is subjected to a signal processing by a video processor, and then displayed on the monitor as an endoscope image.

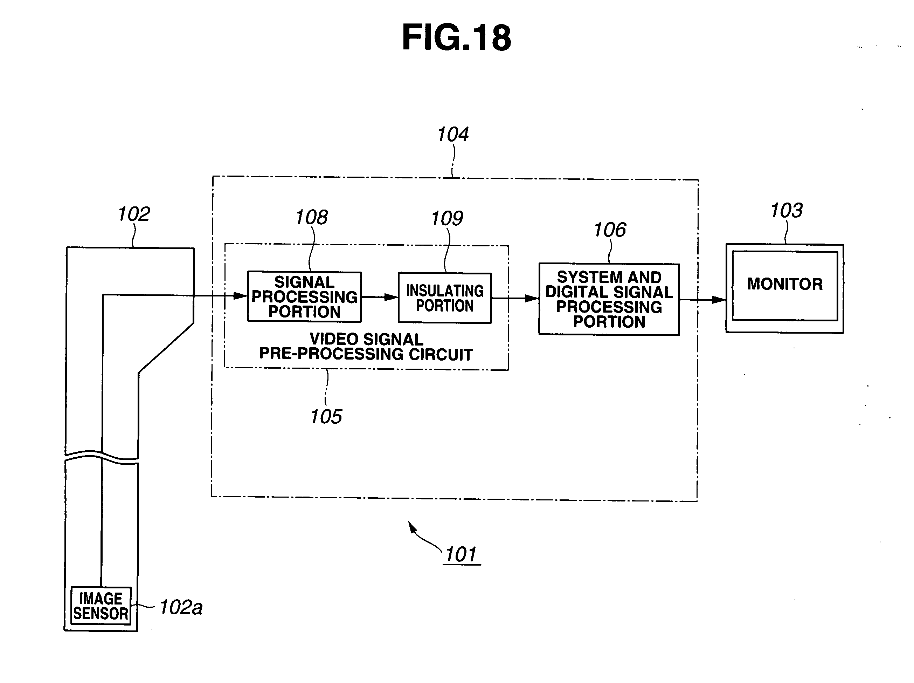

Now, a conventional electronic endoscope system will be described with reference to Figs. 18 to 20.

As shown in Fig. 18, a conventional electronic endoscope system 101 includes an electronic endoscope 102, a video processor 104, and a monitor 103.

The electronic endoscope 102 includes an image sensor (CCD, C-MOS sensor, or the like) 102a as image pickup means inside a distal end of an insertion portion that can be inserted into a lumen.

An image pickup signal from the image sensor 102a is inputted to a video signal pre-processing circuit 105 in the video processor 104. In the video signal pre-processing circuit 105, a signal processing portion 108 performs sampling, noise removal, white balance, and A/D conversion processings of the image pickup signal from the image sensor 102a.

The video signal pre-processing circuit 105 transmits the video signal processed by the signal processing portion 108 to a system and digital signal processing portion 106 in a post-stage while being electrically insulated by an insulating portion 109 constituted by, for example, a photocoupler.

The system and digital signal processing portion 106 is constituted by a general personal computer (PC) board, performs various digital processings such as color tone, enhancing, color conversion, or character superimposing processings of the digital video signal processed by the signal processing portion 108, and performs an image output processing for outputting a desired image to the monitor 103.

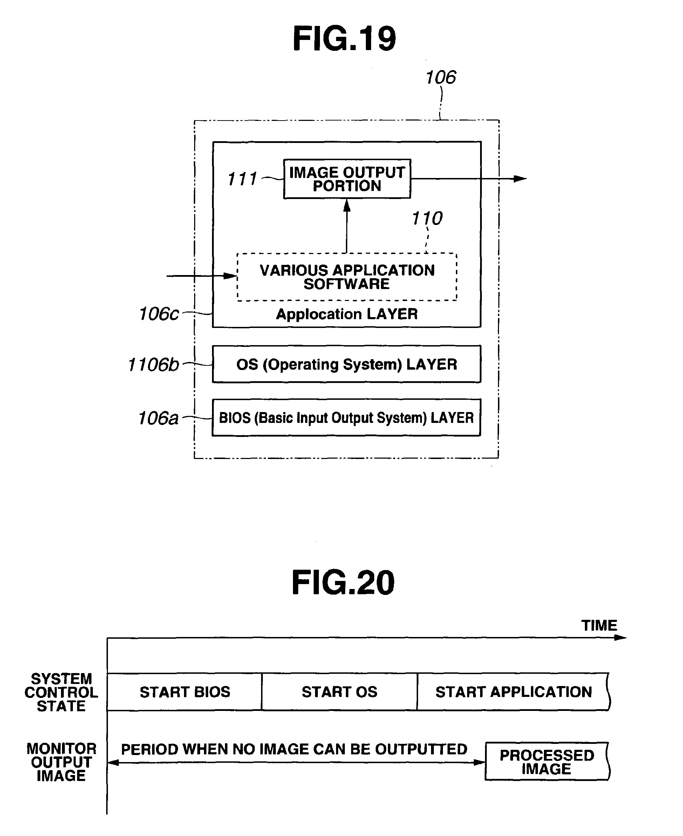

Software of the system and digital signal processing portion 106 has a hierarchical structure including a BIOS (Basic Input Output System) layer 106a, an OS (Operating System) layer 106b, an Application layer 106c and the like, as shown in Fig. 19 as in a general personal computer (PC).

The Application layer 106c includes various application software portions 110 that perform various digital processings such as color tone, enhancing, color conversion, or character superimposing processings, and an image output portion 111 that performs the image output processing for outputting a desired image to the monitor 103.

However, in the video processor 104 in the conventional electronic endoscope system 101 in Fig. 18, the image from the image output portion 111 is subjected to various processings by desired application software portions 110 in the Application layer 106c after starts of a BIOS in the BIOS layer 106a and an OS in the OS layer 106b as shown in Fig. 20.

Further, the image is outputted to the monitor 103 by the image output processing by the image output portion 111, and thus a predetermined sequence including the starts of the BIOS, OS and application needs to be passed to start display on the monitor 103.

Thus, the endoscope image cannot be displayed on the monitor 103 immediately after power-on of the video processor 104. Specifically, in the conventional example, there is a defect that it takes time for the monitor 103 to display the endoscope image after the power-on of the video processor 104.

In the conventional electronic endoscope system 101 in which observation images are generated on software started by the system and digital signal processing portion 106 constituted by the PC board, a case where the software hangs up is supposed.

To address the case, the system and digital signal processing portion 106 constituted by the PC board needs to be restarted. For a restart, there is a period when the observation images cannot be displayed at the restart of the system, which may prevent quick endoscopy.

The present invention is achieved in view of the above described circumstances, and has an object to provide an endoscope apparatus that can output an endoscope image based on an image pickup signal of an endoscope to a display device even if software is not started when a video signal is generated using the software.

### Disclosure of Invention

### Means for Solving the Problem

An endoscope apparatus according to the present invention includes: a first video signal generating portion that generates a first video signal displayed on a display device as an endoscope image from an image pickup signal picked up by an image pickup device of an endoscope; a second video signal generating portion that performs a signal processing of the first video signal using application software that runs on an operating system and generates a second video signal;
a video signal selecting portion that selects one of the first video signal and the second video signal and outputs the video signal to the display device; and
a control portion that determines or estimates whether the second signal generating portion can output the second video signal, and applies a control signal so that the video signal selecting portion outputs the first video signal when determining or estimating that the second video signal generating portion cannot output the second video signal.

### Brief Description of the Drawings

Fig. 1 is a block diagram which shows a configuration of an electronic endoscope system according to Embodiment 1 of the present invention;
Fig. 2 is a diagram which shows a configuration of software of a system and digital signal processing portion in Fig. 1;
Fig. 3 is a flowchart illustrating a flow of processings of a video processor in Fig. 1;
Fig. 4 is a diagram which shows a through image displayed on a monitor in the processings in Fig. 2;
Fig. 5 is a diagram which shows a processed image displayed on the monitor in the processings in Fig. 2;
Fig. 6 is a timing chart illustrating the processings in Fig. 2;
Fig. 7 is a diagram which shows a configuration of software of a variant of the system and digital signal processing portion in Fig. 1;
Fig. 8 is a block diagram which shows a configuration of a first variant of the electronic endoscope system in Fig. 1;
Fig. 9 is a block diagram which shows a configuration of a second variant of the electronic endoscope system in Fig. 1;
Fig. 10 is a diagram which shows a configuration of a display panel in Fig. 8;
Fig. 11 is a block diagram which shows a configuration of a third variant of the electronic endoscope system in Fig. 1;
Fig. 12 is a diagram which illustrates an operation of a warning information superimposing portion in Fig. 11;
Fig. 13 is a block diagram which shows a configuration of a fourth variant of the electronic endoscope system in Fig. 1;
Fig. 14 is a flowchart illustrating a flow of processings of a video processor in Fig. 13;
Fig. 15 is a block diagram which shows a configuration of an electronic endoscope system according to Embodiment 2 of the present invention;
Fig. 16 is a flowchart illustrating a flow of processings of a video processor in Fig. 15;
Fig. 17 is a timing chart illustrating the processings in Fig. 16;
Fig. 18 is a block diagram which shows a configuration of a conventional electronic endoscope system;
Fig. 19 is a diagram which shows a configuration of software of a system and digital signal processing portion in Fig. 18; and
Fig. 20 is a timing chart illustrating an image output processing by the system and digital signal processing portion in Fig. 18.

### Best Mode for Carrying Out the Invention

Now, embodiments of the present invention will be described with reference to the drawings.

### (Embodiment 1)

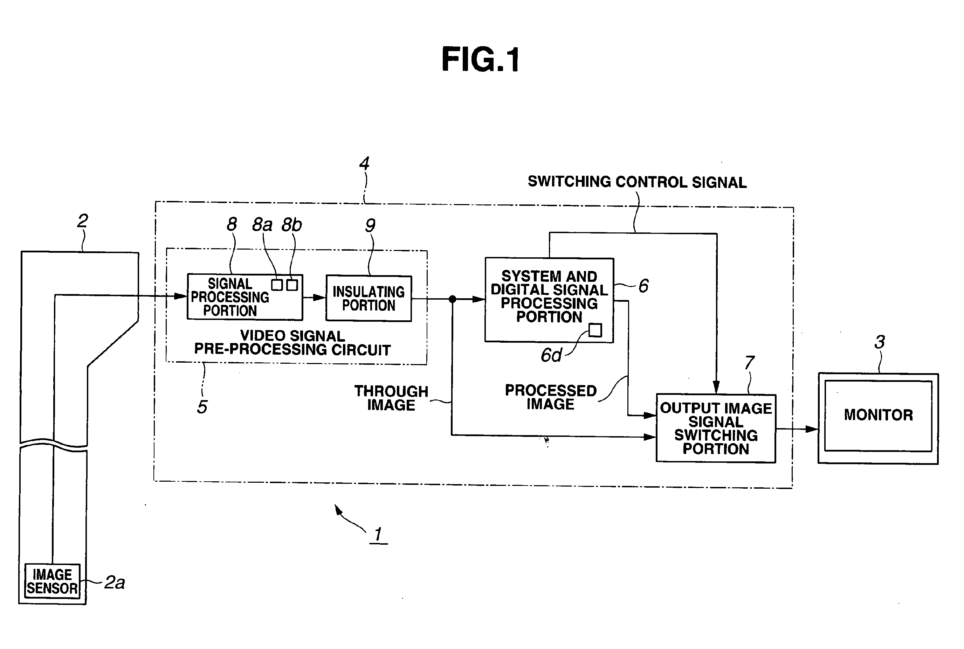

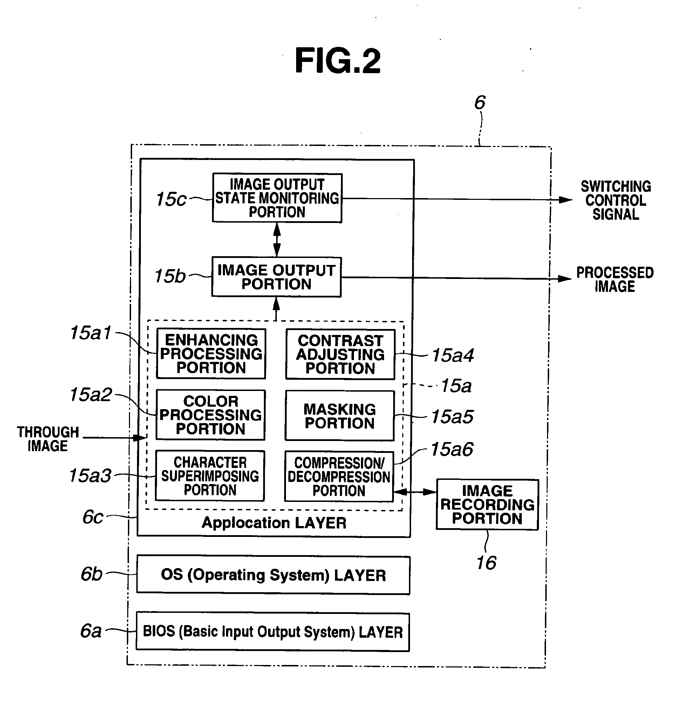

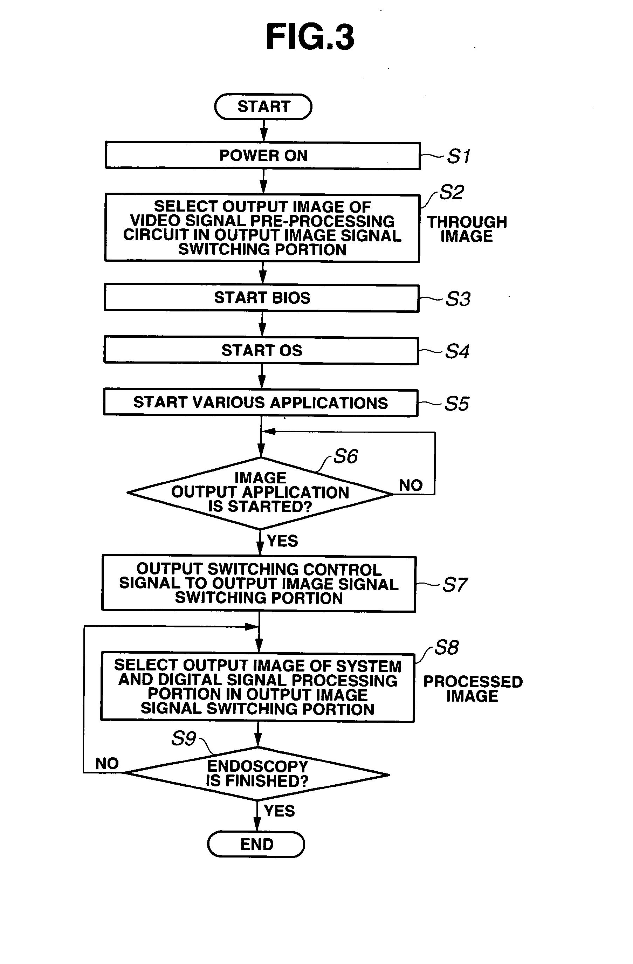

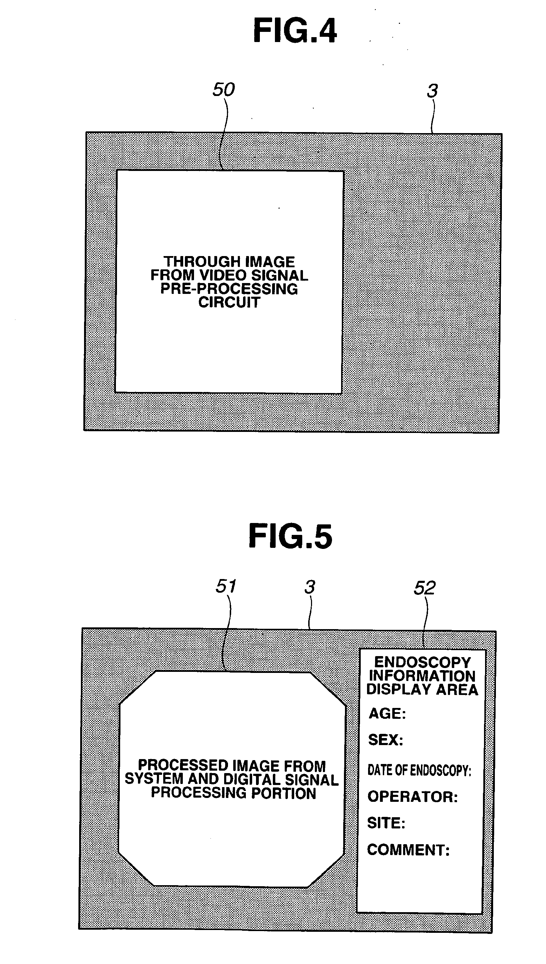

Figs. 1 to 14 relate to Embodiment 1 of the present invention, Fig. 1 is a block diagram which shows a configuration of an electronic endoscope system, Fig. 2 is a diagram which shows a configuration of software of a system and digital signal processing portion in Fig. 1, Fig. 3 is a flowchart illustrating a flow of processings of a video processor in Fig. 1, Fig. 4 is a diagram which shows a through image as an endoscope image displayed on a monitor in the processings in Fig. 2, and Fig. 5 is a diagram which shows a processed image as an endoscope image displayed on the monitor in the processings in Fig. 2.

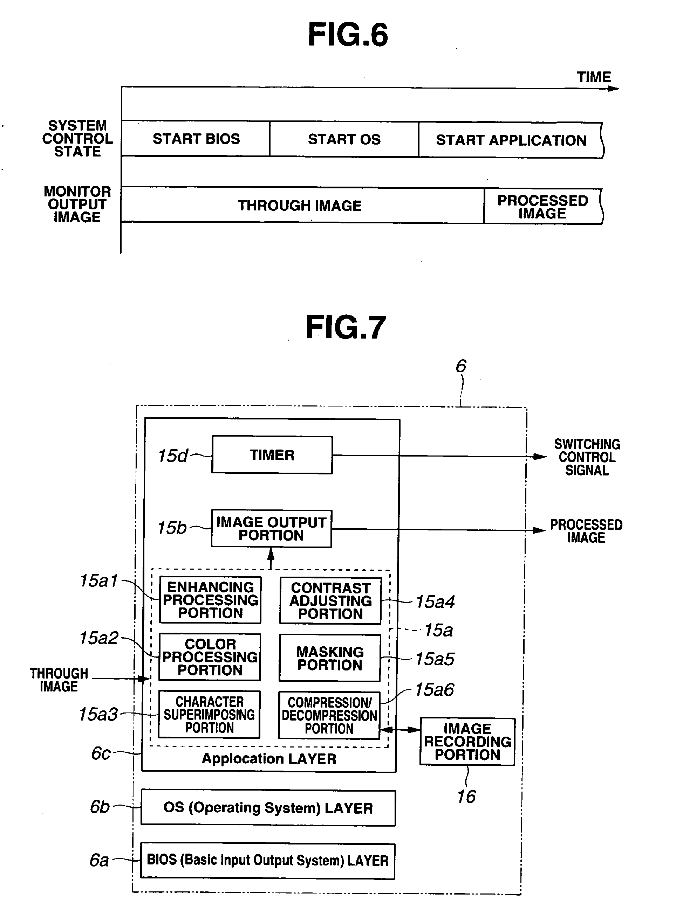

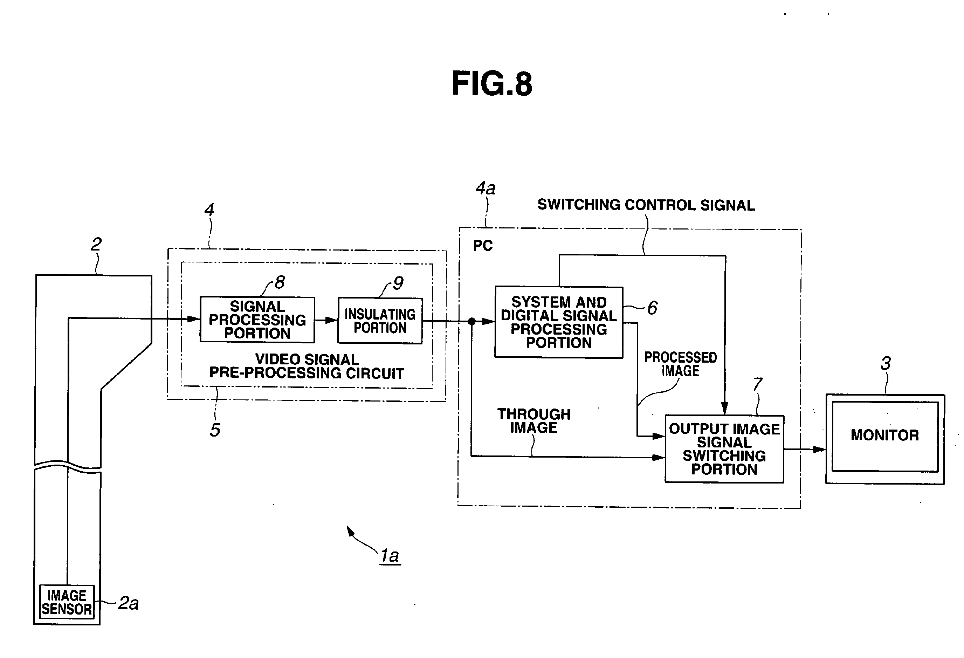

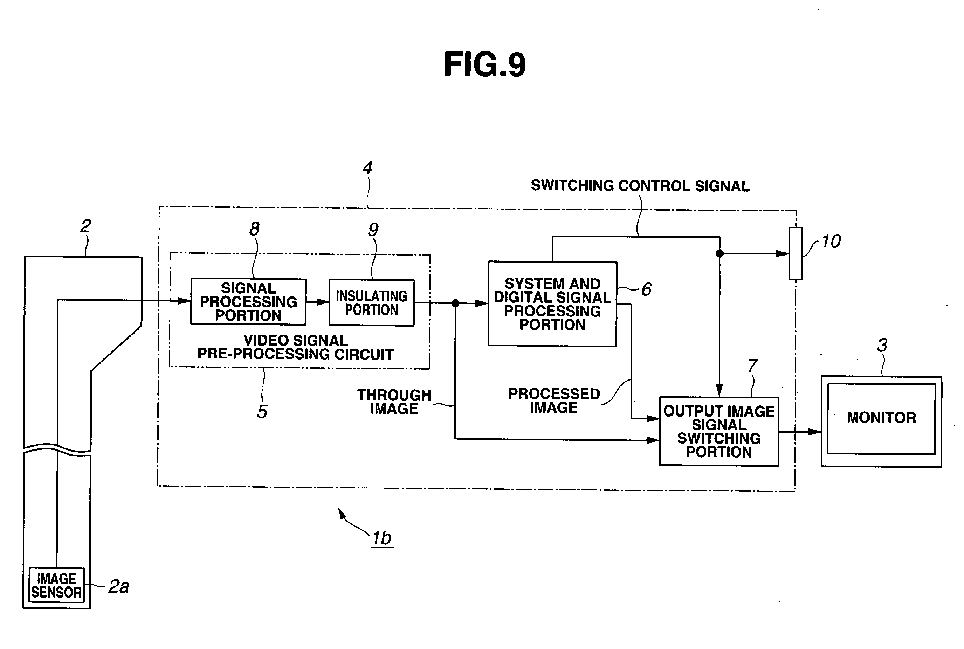

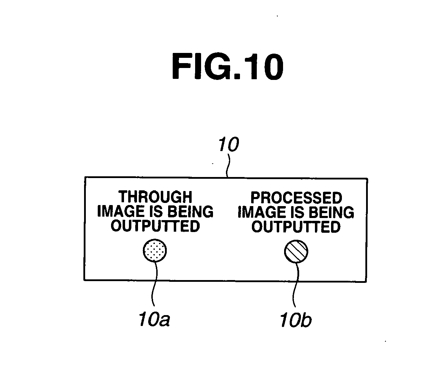

Fig. 6 is a timing chart illustrating the processings in Fig. 2, Fig. 7 is a diagram which shows a configuration of software of a variant of the system and digital signal processing portion in Fig. 1, Fig. 8 is a block diagram which shows a configuration of a first variant of the electronic endoscope system in Fig. 1, Fig. 9 is a block diagram which shows a configuration of a second variant of the electronic endoscope system in Fig. 1, and Fig. 10 is a diagram which shows a configuration of a display panel in Fig. 8.

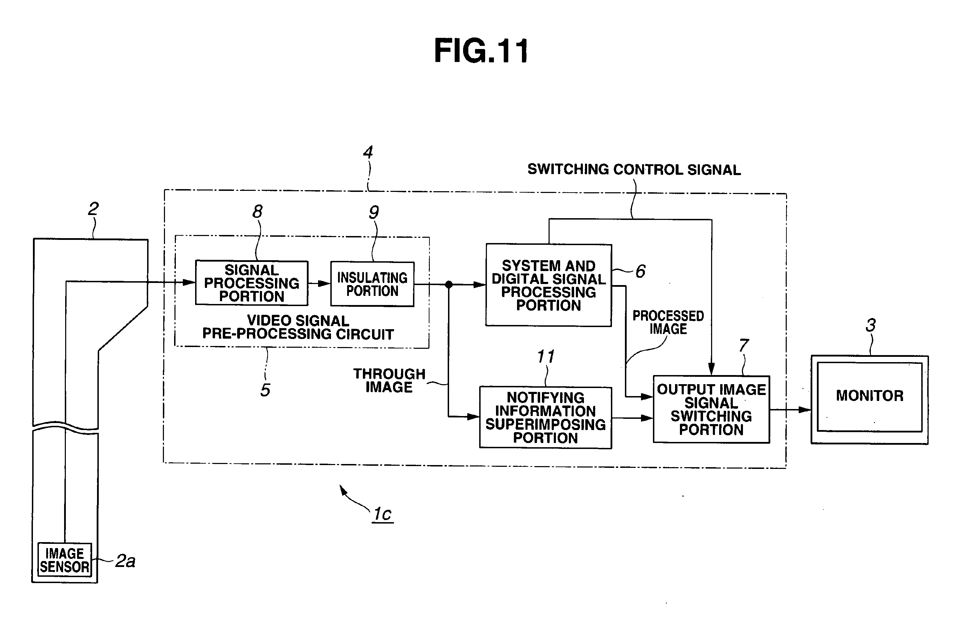

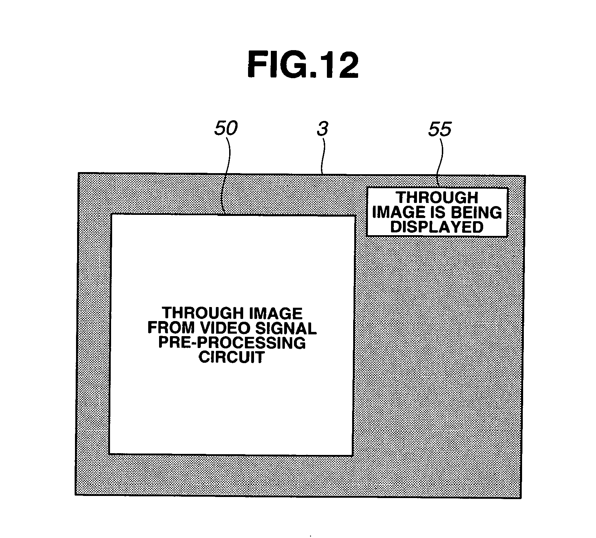

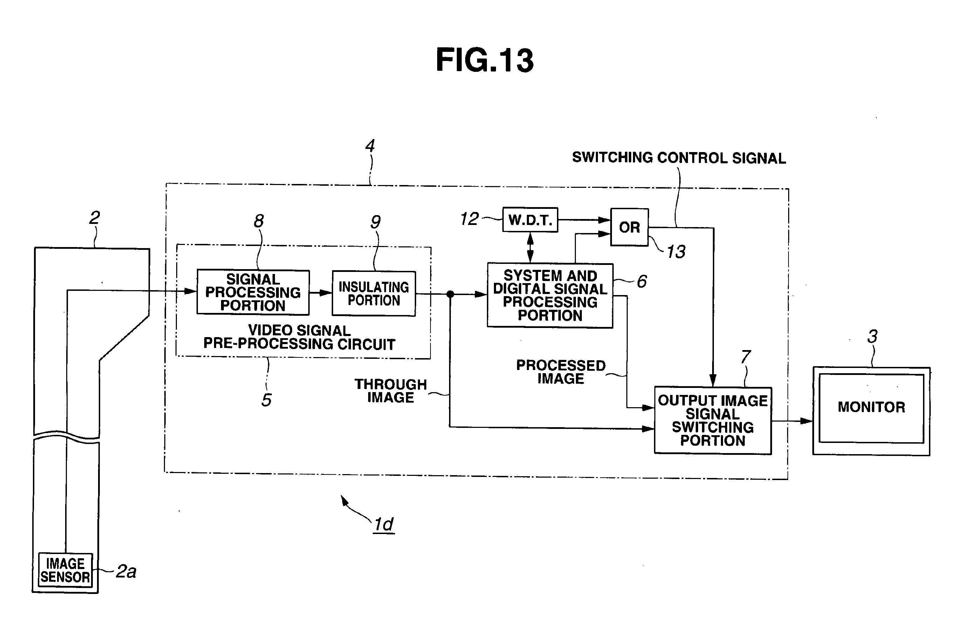

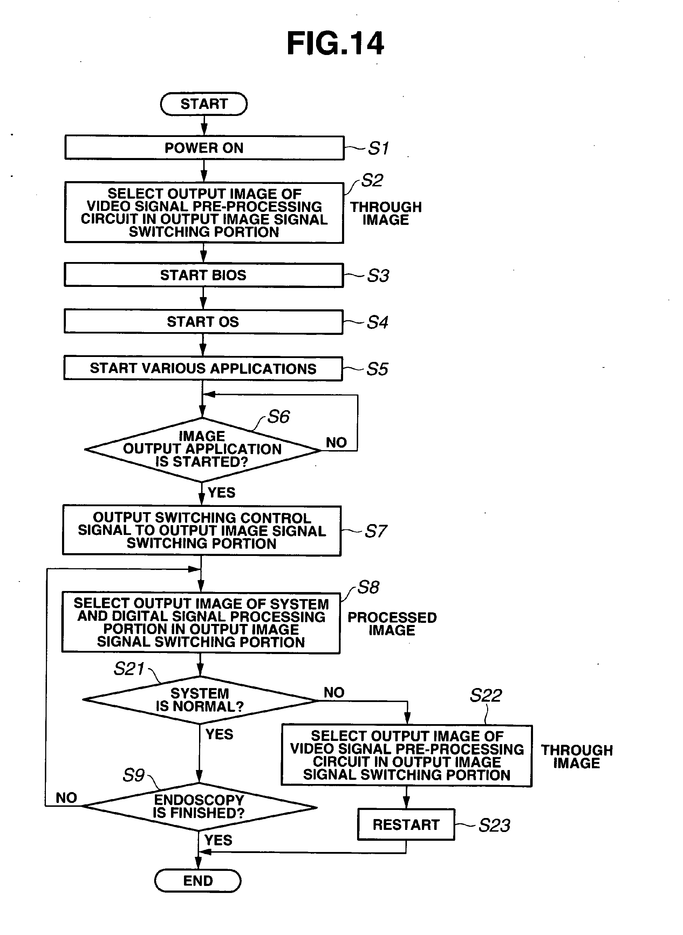

Fig. 11 is a block diagram which shows a configuration of a third variant of the electronic endoscope system in Fig. 1, Fig. 12 is a diagram which illustrates an operation of a warning information superimposing portion in Fig. 11, Fig. 13 is a block diagram which shows a configuration of a fourth variant of the electronic endoscope system in Fig. 1, and Fig. 14 is a flowchart illustrating a flow of processings of a video processor in Fig. 13.

As shown in Fig. 1, an electronic endoscope system 1 that constitutes an endoscope apparatus includes an electronic endoscope 2 including an image pickup device, a video processor 4 that performs a signal processing, and a monitor 3 that displays an endoscope image.

The electronic endoscope 2 includes an elongated insertion portion 2b that can be inserted into a body cavity of a patient, and an image sensor (CCD, C-MOS sensor, or the like) 2a as the image pickup device is provided in a distal end portion of the insertion portion 2b.

An image pickup signal picked up by the image sensor 2a is inputted to a video signal pre-processing circuit 5 as first video signal generating means in the video processor 4 that performs a signal processing for the image sensor 2a.

The video signal pre-processing circuit 5 includes a signal processing portion 8 and an insulating portion 9. The signal processing portion 8 includes a CDS circuit 8a that performs a correlative double sampling processing (abbreviated as CDS) of the image pickup signal from the image sensor 2a, and an A/D conversion circuit 8b and the like that perform noise removal, white balance, and A/D conversion processings of the image pickup signal from the image sensor 2a.

The signal processing portion 8 in the video signal pre-processing circuit 5 performs a basic signal processing of the image pickup signal corresponding to an image picked up by the image sensor 2a, the signal processing being required for displaying the image picked up by the image sensor 2a as an endoscope image on the monitor 3 as display means.

The signal processing portion 8 in the video signal pre-processing circuit 5 enters an operating state in a shorter time than a system and digital signal processing portion 6 described later and outputs a video signal when the electronic endoscope system 1 is powered on from off.

Then, the video signal is transmitted to the system and digital signal processing portion 6 as second video signal generating means in a post-stage via an insulating portion 9 constituted by, for example, a photocoupler. In this case, the insulating portion 9 electrically insulates the signal processing portion 8 electrically connected to the image sensor 2a inserted into a body of a patient, from the system and digital signal processing portion 6.

The system and digital signal processing portion 6 is constituted by a personal computer (PC) board including an MPU (microprocessor) 6d.

The system and digital signal processing portion 6 performs various digital signal processings (also referred to as digital processings) of a digital video signal generated by the basic signal processing by the signal processing portion 8 in a pre-stage.

Specifically, the system and digital signal processing portion 6 performs various digital processings such as color tone, enhancing, color conversion, scaling, or gamma correction processings of the digital video signal outputted from the video signal pre-processing circuit 5.

The system and digital signal processing portion 6 outputs the video signal (also referred to as an image signal) subjected to the various digital processings to the monitor 3 via an output image signal switching portion 7 as video signal selecting means.

The output image signal switching portion 7 selectively outputs, to the monitor 3, an image signal (hereinafter referred to as a processed image) subjected to the digital processing from the system and digital signal processing portion 6, and an image signal (hereinafter referred to as a through image) before the digital processing outputted from the insulating portion 9 in the video signal pre-processing circuit 5.

The output image signal switching portion 7 selects one of the processed image and the through image and outputs the image to the monitor 3 by a switching control signal from the system and digital signal processing portion 6.

The output image signal switching portion 7 selects and outputs the through image when the switching control signal from the system and digital signal processing portion 6 is, for example, a High level.

Software of the system and digital signal processing portion 6 has a hierarchical structure including a BIOS (Basic Input Output System) layer 6a, an OS (Operating System) layer 6b, and an Application layer 6c as shown in Fig. 2 as in a general personal computer (PC).

The Application layer 6c includes an application software portion 15a that performs the digital processing of the through image from the insulating portion 9 in the video signal pre-processing circuit 5 and generates the processed image, an image output portion 15b that performs an image output processing for outputting the processed image to the monitor 3, and an image output state monitoring portion 15c as processing state determination means that monitors a processing state of the image output processing by the image output portion 15b and outputs the switching control signal to the output image signal switching portion 7.

The application software portion 15a includes an enhancing processing portion 15a1 that performs an enhancing processing, a color processing portion 15a2 that performs color tone and color conversion processings, a character superimposing portion 15a3 that performs a superimposing processing of character information, a contrast adjusting portion 15a4 that performs a contrast processing of the image, and a masking portion 15a5 that performs a masking processing of the image.

The application software portion 15a also includes a compression/decompression portion 15a6 that performs a digital compression processing of the image, records the image in the image recording portion 16, reads the image subjected to the digital compression processing from the image recording portion 16, and performs a digital decompression processing.

The image recording portion 16 may be placed inside or outside the system and digital signal processing portion 6.

The image output state monitoring portion 15c as the processing state determination means determines whether the state of the processing by the image output processing portion 15b for outputting the processed image, which is a final digital processing for generating the processed image in the application software portion 15a, is a completed state.

When it is determined that the processing state is the completed state, the image output state monitoring portion 15c changes the switching control signal from a High level to a Low level, and switches the image from the through image to the processed image by the switching control signal at the Low level.

The embodiment having such a configuration includes the video signal pre-processing circuit 5 that generates a first video signal displayed as an endoscope image (through image) on the monitor 3 as a display device, and the system and digital signal processing portion 6 that performs a signal processing of the first video signal using application software that runs on an operating system, and generates a second video signal displayed as an endoscope image (processed image) on the monitor 3.

The image output state monitoring portion 15c monitors whether the system and digital signal processing portion 6 can output the processed image to the monitor 3, and controls the output image signal switching portion 7 by the switching control signal so as to output the through image to the monitor 3 when the system and digital signal processing portion 6 cannot output the processed image.

Thus, for example, even if the system and digital signal processing portion 6 has not entered a start state for performing the signal processing when the video processor 4 is powered on, the video processor 4 outputs the first video signal by the video signal pre-processing circuit 5 to the monitor 3. On the monitor 3, the through image corresponding to the image picked up by the image pickup device can be displayed.

Specifically, even if there is a period when the system and digital signal processing portion 6 cannot generate the processed image, the through image is displayed in the period. This nearly eliminates a period when no endoscope image is displayed on the monitor.

Next, an operation of the present embodiment will be described with reference to the flowchart in Fig. 3 and Figs. 4 to 6.

As shown in Fig. 3, in Step S1, the electronic endoscope 2 and the monitor 3 are connected to the video processor 4, and the electronic endoscope system 1 is powered on. Then, in Step S2, the system and digital signal processing portion 6 in the video processor 4 controls the output image signal switching portion 7 so as to output the through image from the insulating portion 9 in the video signal pre-processing circuit 5 to the monitor 3 by the switching control signal at the High level.

In the processing immediately after the electronic endoscope system 1 is powered on, that is, in Step S2, the system and digital signal processing portion 6 is in a state before a start of a BIOS. Then, the system and digital signal processing portion 6 outputs the switching control signal to the output image signal switching portion 7 as a default first status signal, for example, at a High level.

By the first status signal, the output image signal switching portion 7 outputs the through image from the insulating portion 9 in the video signal pre-processing circuit 5 to the monitor 3. Fig. 4 shows an example of a through image 50 displayed on the monitor 3.

In this case, the image picked up by the image sensor 2a is displayed as an endoscope image. In this case, the masking processing by the masking portion 15a5 is not performed, and thus, for example, an image picked up by an image sensor 2a having a square image pickup surface is displayed as an endoscope image. Specifically, the endoscope image is displayed with an outline corresponding to an outline of the image pickup surface.

Next, the system and digital signal processing portion 6 in the video processor 4 performs a start processing of the BIOS in the BIOS (Basic Input Output System) layer 6a in Step S3, and performs a start processing of an OS in the OS (Operating System) layer 6b in Step S4. Further, the system and digital signal processing portion 6 starts various application software in the Application layer 6c in Step S5.

Then, in the system and digital signal processing portion 6, the image output state monitoring portion 15c in the Application layer 6c determines whether an application for the image output processing by the image output portion 15b is started in Step S6.

When the image output state monitoring portion 15c determines that the application for the image output processing by the image output portion 15b is started, the system and digital signal processing portion 6 outputs the switching control signal to the output image signal switching portion 7 as a second status signal, for example, at a Low level in Step S7.

Then, the output image signal switching portion 7 outputs the processed image subjected to the digital processing from the system and digital signal processing portion 6 to the monitor 3 by the first status signal in Step S8.

Fig. 5 shows an example of a processed image 51 displayed on the monitor 3. As shown in Fig. 5, for the processed image 51, character information such as endoscopy information is displayed in a superimposing manner on an endoscopy information display area 52 besides the image.

In this case, the masking processing by the masking portion 15a5 is performed, and thus, for example, four corners of the image picked up by the image sensor 2a having the square image pickup surface are masked, and an endoscope image having a substantially octagonal outline is displayed.

Then, the video processor 4 repeats the processing in Step S8 and continuously outputs the processed image subjected to the digital processing from the system and digital signal processing portion 6 to the monitor 3 until a finish of the endoscopy is confirmed in Step S9.

As such, in the present embodiment, as shown in Fig. 6, during the start of the BIOS immediately after the electronic endoscope system 1 is powered on, during the start of the OS, and until immediately before the start of the image output processing, the through image from the insulating portion 9 in the video signal pre-processing circuit 5 is displayed on the monitor 3.

Then, after the start of the image output processing, the processed image subjected to the digital processing from the system and digital signal processing portion 6 is displayed on the monitor 3.

Thus, in the present embodiment, the through image can be displayed on the monitor 3 immediately after the electronic endoscope system 1 is powered on. Irrespective of the start state of a digital video processing circuit that generates the endoscope image by the digital processing, an operator can continuously observe the inside of the body cavity by an endoscope image (through image or processed image) as an observation image based on the image pickup signal of the endoscope.

In the present embodiment, the switching control signal is outputted to the output image signal switching portion 7 on the basis of a monitoring result of the image output state monitoring portion 15c, but not limited thereto.

For example, as shown in Fig. 7, a timer 15d may be provided instead of the image output state monitoring portion 15c so as to measure a time required between the power-on and the start of the image output processing to estimate whether the image output state is entered.

In a period shorter than the time required until the start of the image output processing by the timer 15d, the timer 15d outputs a switching control signal as the first status signal to the output image signal switching portion 7.

On the other hand, the timer 15d may output a switching control signal as the second status signal to the output image signal switching portion 7 in a period longer than the time required until the start of the image output processing.

The timer 15d may be added to the configuration in Fig. 2 so as to perform margin period measurement of variations due to delays in starts of the system and application, thereby allowing the through image to be displayed as an observation image (endoscope image) in accordance with processed image generation based on the delays.

Specifically, when there is a possibility of delays in processed image generation, the timer 15d measures a margin period, and the image is switched from the through image to the processed image after the margin period, thereby allowing an observation image to be continuously displayed on the monitor 3.

In Fig. 1 or the like of the present embodiment, the video signal pre-processing circuit 5, the system and digital signal processing portion 6, and the output image signal switching portion 7 are provided in the video processor 4.

However, not being limited thereto, as shown in Fig. 8, the same operation and effect as in the present embodiment can be obtained when the video signal pre-processing circuit 5 is provided in the video processor 4, and the system and digital signal processing portion 6 and the output image signal switching portion 7 are constituted by a personal computer (PC) 4a.

Specifically, the video signal pre-processing circuit 5, and the system and digital signal processing portion 6 and the output image signal switching portion 7 may be provided in separate casings.

As shown in Fig. 9, a display panel 10 as a notifying portion is provided in the video processor 4, and as shown in Fig. 10, lighting of LEDs 10a and 10b on the display panel 10 is controlled by a switching control signal. A user may be notified whether the image displayed on the monitor 3 is a through image or a processed image in this manner.

Similarly, as shown in Fig. 11, a notifying information superimposing portion 11 that superimposes a notifying message on the through image may be provided in the video processor 4, and display a notifying display area 51 adjacent to the through image 50 to notify the user of the through image 50 being displayed on the monitor 3 as shown in Fig. 12. Notification by sound may be allowed.

Also, as shown in Fig. 13, a watchdog timer (W.D.T.) 12 that monitors a control state of the system and digital signal processing portion 6 and an OR circuit 13 may be provided so that the through image can be displayed on the monitor 3 as the observation image when the system and digital signal processing portion 6 hangs up.

Specifically, in the configuration in Fig. 13, in the video processor 4, the W.D.T. 12 determines whether the system of the system and digital signal processing portion 6 is normally operating in Step S21 after the processing in Step S8 as shown in Fig. 14.

When it is determined that there is an abnormality in the system of the system and digital signal processing portion 6, the output image signal switching portion 7 outputs the through image from the insulating portion 9 in the video signal pre-processing circuit 5 to the monitor 3 in Step S22.

In this case, as in Step S23, the W.D.T. 12 may restart (reset) the system and digital signal processing portion 6. Then, the processing may be finished or return to Step S2.

Thus, with the configuration in Fig. 13, in addition to the advantage of the present embodiment, the through image can be displayed as the observation image even if there is an abnormality in the system of the system and digital signal processing portion 6, and also the through image can be displayed as the observation image in a restart period of the system and digital signal processing portion 6.

### (Embodiment 2)

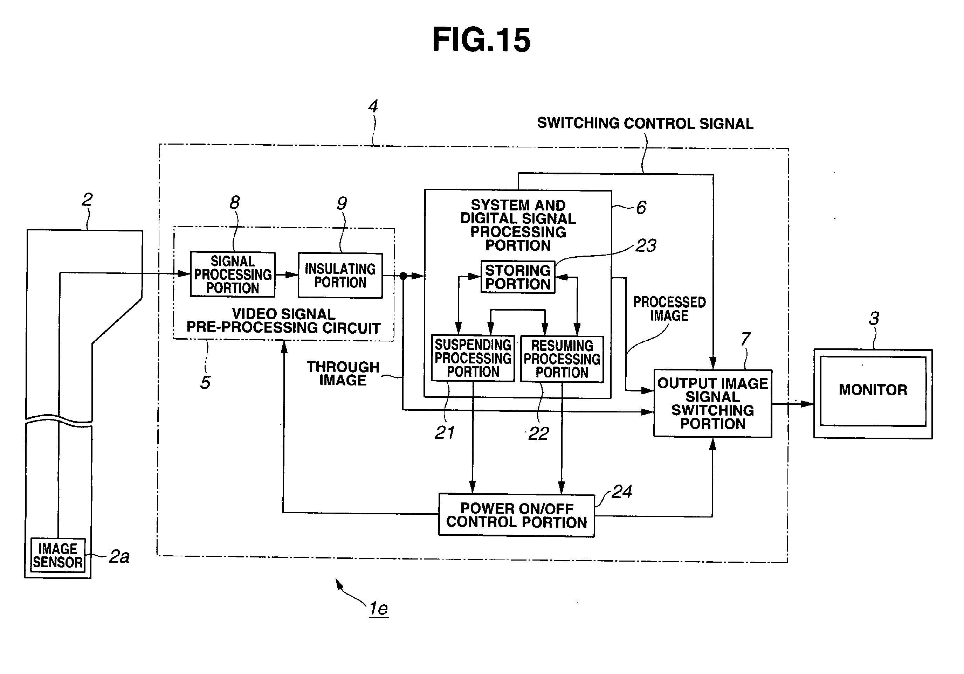

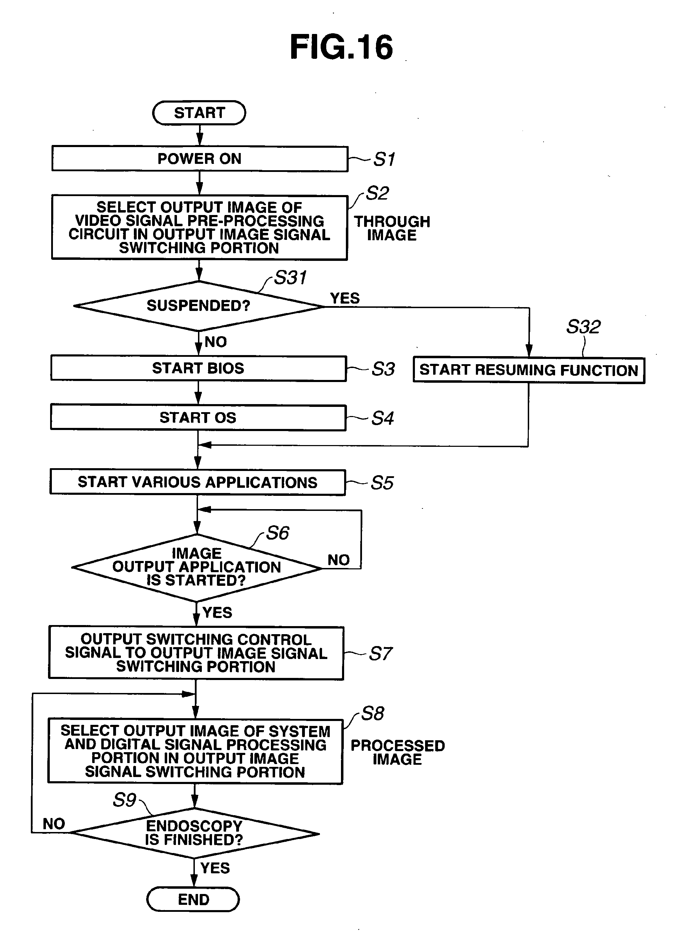

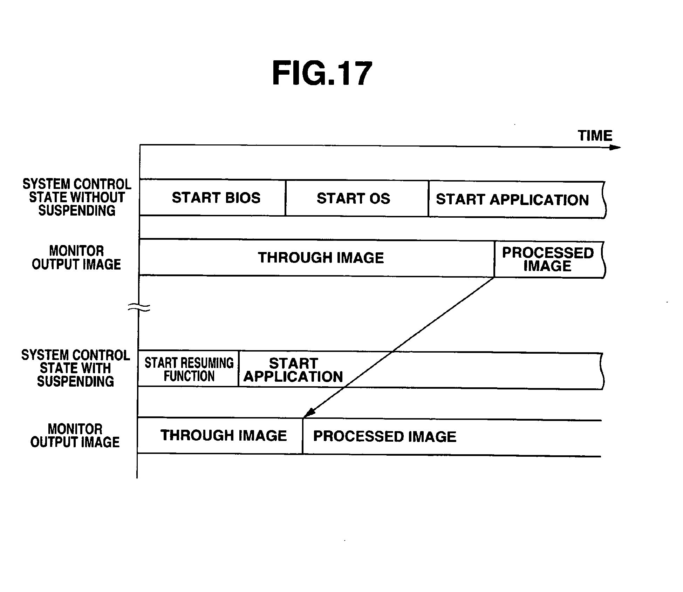

Figs. 15 to 17 relate to Embodiment 2 of the present invention, Fig. 15 is a block diagram which shows a configuration of an electronic endoscope system, Fig. 16 is a flowchart illustrating a flow of processings of a video processor in Fig. 15, and Fig. 17 is a timing chart illustrating the processings in Fig. 16.

Embodiment 2 is substantially the same as Embodiment 1, and thus differences only will be described, and the same components are denoted by the same reference numerals and descriptions thereof will be omitted.

A system and digital signal processing portion 6 of the present embodiment includes, as shown in Fig. 15, a suspending processing portion 21 that suspends an operation of a system, a storing portion 23 that stores processing information of the system stopped by the suspending processing portion 21, and a resuming processing portion 22 that resumes a processing on the basis of the processing information of the system stored in the storing portion 23. Other configurations are the same as in Embodiment 1.

In the present embodiment thus configured, as shown in Fig. 16, a video processor 4 determines whether the system and digital signal processing portion 6 is suspended by the suspending processing portion 21 in Step S31 after a processing in Step S2. When the system and digital signal processing portion 6 is not suspended, the process proceeds to Step S3, and when the system and digital signal processing portion 6 is suspended, the process proceeds to Step S32.

In Step S32, the video processor 4 read a suspended processing from the processing information stored in the storing portion 23, the resuming processing portion 22 returns the processing of the system and digital signal processing portion 6 to the processing before suspending, and the process proceeds to Step S5. Other operations are the same as in Embodiment 1.

Thus, in the present embodiment, in addition to the advantage of Embodiment 1, when the resuming processing portion 22 returns the suspended system and digital signal processing portion 6 to a normal processing state, a through image in a short time can be displayed on a monitor 3 as an observation image, and the observation image can be continuously and quickly switched to a processed image as shown in Fig. 17.

The present invention is not limited to the above described embodiments, and various changes and modifications may be made without changing the gist of the present invention.

### Industrial Applicability

The first video signal displayed on the display device by a basic signal processing of the image pickup signal of the image pickup device, and the second video signal generated from the first video signal using the software can be switched and outputted to the display device, and the first video signal is outputted to the display device when the second video signal cannot be outputted.

Even when it takes time to generate the second video signal after the start of the software as at the time of power-on, the first video signal can be outputted to the display device and the image picked up by the image pickup device can be displayed as the endoscope image, thereby significantly reducing time when no endoscope image is displayed. Also, an endoscope apparatus is achieved that allows an operator easy observation and diagnosis.

The present application is filed claiming the priority of Japanese Patent Application No. 2006-247168 filed in Japan on September 12, 2006, and the disclosure thereof is incorporated into the specification, claims, and drawings of the present application.

## Claims

1. An endoscope apparatus comprising:
a first video signal generating portion that generates a first video signal displayed on a display device as an endoscope image from an image pickup signal picked up by an image pickup device of an endoscope;
a second video signal generating portion that performs a signal processing of the first video signal using application software that runs on an operating system and generates a second video signal;
a video signal selecting portion that selects one of the first video signal and the second video signal and outputs the signal to the display device; and
a control portion that determines or estimates whether the second signal generating portion can output the second video signal, and applies a control signal so that the video signal selecting portion outputs the first video signal when determining or estimating that the second signal generating portion cannot output the second video signal.

2. The endoscope apparatus according to claim 1, wherein the control portion monitors whether an output portion connected to an output end of the second signal generating portion that outputs the second video signal can output the second signal, and thus generates the control signal.

3. The endoscope apparatus according to claim 1, wherein the control portion is constituted by a timer that outputs, as the control signal, a determination signal indicating whether a time has passed required between the time when the second video signal generating portion is powered on from off and the time when the operating system is started and an image processing in accordance with the application software is started.

4. The endoscope apparatus according to claim 1, wherein the control portion is constituted by an operation state monitoring portion that temporally monitors whether the second video signal generating portion is in a normal operation state, and thus outputs, as the control signal, a determination signal indicating whether the second video signal generating portion is in the normal operation state.

5. The endoscope apparatus according to claim 1, wherein the control portion controls to temporally monitor whether the second video signal generating portion is in a normal operation state, and applies, as the control signal, a determination signal determining that the second video signal generating portion is not in the normal operation state to the video signal selecting portion, and cause the video signal selecting portion to output the first video signal, and controls to restart the second video signal generating portion.

6. The endoscope apparatus according to claim 1, further comprising a notifying portion that notifies a user which of the first video signal and the second video signal is selected by the video signal selecting portion.

7. The endoscope apparatus according to claim 1, wherein the second video signal generating portion includes a suspending processing portion that suspends a processing operation including a signal processing for generating the second video signal, an operation information storing portion that stores information on the processing operation suspended by the suspending processing portion, and a resuming processing portion that returns to the processing operation suspended by the suspending processing portion on the basis of the information on the processing operation stored by the operation information storing portion.

8. The endoscope apparatus according to claim 1, wherein the first video signal generating portion and the second video signal generating portion are housed in one casing.

9. The endoscope apparatus according to claim 1, wherein the first video signal generating portion and the second video signal generating portion are housed in separate casings.

10. The endoscope apparatus according to claim 1, wherein the first video signal generating portion and the second video signal generating portion are electrically insulated by an insulating portion.

11. The endoscope apparatus according to claim 1, wherein the second video signal generating portion is constituted by a personal computer board including a microprocessor that performs a signal processing in accordance with application software that operates after starts of a BIOS (Basic Input Output System) and the operating system.

12. The endoscope apparatus according to claim 1, wherein the second video signal generating portion performs a signal processing of at least one of an enhancing processing portion that performs an enhancing processing, a color processing portion that performs color tone and color conversion processings, a character superimposing portion that performs a superimposing processing of character information, a contrast adjusting portion that performs a contrast processing of the image, and a masking portion that performs a masking processing of the image.

13. The endoscope apparatus according to claim 1, wherein the second video signal generating portion includes a recording portion that compresses and records the second video signal.

14. The endoscope apparatus according to claim 1, wherein the first video signal generating portion includes a correlative double sampling circuit that performs correlative double sampling of the image pickup signal.

15. The endoscope apparatus according to claim 1, wherein the first video signal generating portion includes a correlative double sampling circuit that performs correlative double sampling of the image pickup signal, and an A/D conversion circuit that converts an output signal of the correlative double sampling circuit from an analog signal to a digital signal.

16. The endoscope apparatus according to claim 15, wherein the second video signal generating portion performs a digital signal processing of a digital first video signal outputted from the first video signal generating portion.

17. The endoscope apparatus according to claim 1, further comprising an electronic endoscope including the image pickup device in a distal end portion of an insertion portion.

18. The endoscope apparatus according to claim 1, further comprising an superimposing portion that superimposes, on an output signal of the first video signal, a signal notifying that the output signal of the first video signal is outputted.

19. The endoscope apparatus according to claim 1, wherein a first endoscope image when the first video signal is displayed on the display device as the endoscope image, and a second endoscope image when the second video signal is displayed on the display device as the endoscope image have different display outlines.

20. The endoscope apparatus according to claim 1, wherein the second video signal generating portion includes a masking processing portion that masks four corners when the first video signal is displayed on the display device as an endoscope image.
